Europäisches Patentamt

European Patent Office

Office européen des brevets

(11) Publication number: **0 318 879**
**A1**

## EUROPEAN PATENT APPLICATION

(21) Application number: 88119760.2

(22) Date of filing: 26.11.88

(51) Int. Cl.⁴: **A61K 39/395** , **C12P 21/00**

A request for correction of the description has been filed pursuant to Rule 88 EPC. A decision on the request will be taken during the proceedings before the Examining Division (Guidelines for Examination in the EPO, A-V, 2.2).

(30) Priority: 03.12.87 US 128556

(43) Date of publication of application:
07.06.89 Bulletin 89/23

(84) Designated Contracting States:
AT BE CH DE ES FR GB GR IT LI LU NL SE

(71) Applicant: **UNIVERSITY HOSPITALS OF CLEVELAND**
**2074 Abington Road**
**Cleveland Ohio 44106(US)**

(72) Inventor: **King, Charles H.**
**3061 Fairfax Road**
**Cleveland Heights, Ohio 44118(US)**
Inventor: **Olds, Richard G.**
**2700 Coventry Road**
**Shaker Heights, Ohio 44120(US)**
Inventor: **Nanduri, Jayasri**
**4147 Greenvale Road nr 104D**
**Cleveland, Ohio, 44121(US)**
Inventor: **Mahmoud, Adel A.F.**
**2 Bratnahl Place**
**Bratnahl, Ohio, 44108(US)**
Inventor: **Lett, Ronald R.**
**4216 Ramsay Crescent**
**Edmonton, Alberta, T6H 5S2(CA)**

(74) Representative: **Patentanwälte Dipl.-Ing. R. Splanemann Dr. B. Reitzner Dipl.-Ing. K. Baronetzky**
**Tal 13**
**D-8000 München 2(DE)**

(54) Vaccine against Schistosoma mansoni.

(57) A monoclonal antibody which identifies the site of the Schistosoma mansoni parasite which elicits in a host a protective response. The molecular antibody binds with a 68,000 molecular weight antigen of Schistosoma mansoni. Such monoclonal antibody can be utilized to produce a vaccine for immunization against Schistosoma mansoni infection, the vaccine comprising substantially pure antigen which identifies the above-described monoclonal antibody.

## VACCINE AGAINST SCHISTOSOMA MANSONI

## BACKGROUND OF THE INVENTION

### Field of the Invention

The present invention relates to a vaccine for immunizing patients against infection by Schistosoma mansoni.

### Background Information

Scores of millions of people around the world are infected by Schistosoma mansoni causing considerable morbidity and mortality and loss of productivity. Recently, safe chemotherapeutic agents have been introduced, but such agents do not have prophylactic properties.

Both epidemiological and experimental data suggest that aquired humoral and cell-mediated (cellular) immunity play significant roles in regulating the intensity of Schistosoma mansoni infection as well as its pathophysiologic sequelae (after effects) (K. S. Warren, "Regulation of the Prevalence and Intensity of Schistosomiasis in Man: Immunology or Ecology", J. Inf. Dis., 127, 595-609, (1973); A. E. Butterworth, D. W. Taylor, M. C. Veith, M. A. Vadas, A. Dussein, R. F. Sturrock and E. Wells, "Studies on the Mechanisms of Immunity in Human Schistosomiasis", Immunol. Rev., 61, 5-39, (1982); S. M. Phillips and D. G. Colley, "Immunologic Aspects of Host Responses to Schistosomiasis: Resistance Immunopathology and Eosinophil Involvement", Prog. Allergy, 24, 49-182, (1978)). It follows that improved control of this trematode (class of parasitic flatworms) parasite may be obtained through the use of immunization to enhance the resistance of individuals to risk for infection (R. M. Anderson, "Transmission Dynamics and Control of Infectious Disease Agents", Population Biology of Infections Diseases, editors: R. M. Anderson and R. M. May, 149- 176, Springer-Verlag, Berlin, (1982)).

In laboratory animals, partial resistance to schistosomiasis has been observed after infection with irradiated (attenuated) cercariae (larval trematode worms) (S. L. James, M. Labine and A. Sher, "Mechanisms of Protective Immunity Against Schistosoma Mansoni Infection in Mice Vaccinated With Irradiated Cercariae, I. Analysis of Antibody and T-lymphocyte Responses in Mouse Strains Developing Differing Levels of Immunity", Cell Immunol., 65, 75-83, (1981); D. A. Dean, D. Murrell, X. Shoutai and B. Mangold, "Immunization of Mice with Ultraviolet Irradiated Schistosoma mansoni Cercariae: A Reevaluation", Am. J. Trop. Med. Hyg., 32, 790-793, (1983)), or after inoculation with crude parasite extracts and/or immunostimulants (R. R. Lett, M. Sc. Thesis, University of Alberta, Edmonton, (1984); S. Horowitz, M. Smolarsky and R. Arnon, "Protection Against Schistosoma mansoni Achieved by Immunization with Sonicated Parasite", Eur. J. Immunol., 12, 327-332, (1982); S. L. James, E. J. Pearce and A. Sher, "Induction of Protective Immunity Against Schistosoma mansoni by a Non-living Vaccine: I. Partial Characterization of Antigens Recognized by Antibodies from Mice Immunized with Soluble Schistosome Extracts", J. Immunol., 134, 3432-3438, (1985); R. Civil, K. S. Warren and A. A. F. Mahmoud, "Conditions for Bacille Calmette-Guerin Induced Resistance to Infection with Schistosoma mansoni in Mice", J. Inf. Dis., 137, 550-555, (1978)). However, parasite material is limited and such methods do not lend themselves to wide-scale field application. Recent studies have shown that limited protection against challenge infection may be conferred by passive immunization using monoclonal antibodies (D. M. Zodda and S. M. Phillips, "Monoclonal Antibody-Mediated Protection Against Schistosoma mansoni Infection in mice", J. Immunol., 129, 2236-2328, (1982); D. A. Harn, M. Mitsuyama and J. R. David, "Anti-Egg Monoclonal Antibodies Protect Against Cercarial Challenge In Vivo", J. Exp. Med., 159, 1371-1387, (1984); M. A. Smith, J. A. Clegg, D. Snary and A. J. Trejdosiewicz, "Passive Immunization of Mice Against Schistosoma mansoni with an IgM Monoclonal Antibody", Parasitology, 84, 83-91, (1983); J. M. Grzych, M. Capron, H. Bazin and A. Capron, "In Vivo and In Vitro Effector Function of IgG2a Monoclonal Anti-S. mansoni Antibodies", J. Immunol., 129, 2739-2743, (1982)).

In two recent studies, it has been reported that active immunization against schistosomiasis has been achieved using purified parasite antigens combined with adjuvants (M. A. Smith and J. A. Clegg, "Vaccination Against Schistosoma mansoni with Purified Surface Antigens", Science, 277, 535-538, (1985);

R. T. Hazdai, F. Levi-Schaffer, V. Brenner, S. Horowitz, Z. Eshhar and R. Arnon, "Protective Monoclonal Antibody Against Schistosoma mansoni: Antigen Isolation, Characterization and Suitability for Active Immunization", J. Immunol., 135, 2772-2780, (1985)). J. M. Grzych, M. Capron, P. H. Lambert, C. Dissous, S. Torres and A. Capron, "An Anti-idiotype Vaccine Against Experimental Schistosomiasis", Nature, 316, 74-76, (1985) describe an anti-idiotype vaccine against schistosomiasis.

J. W. Kazura, H. Cicirello and J. W. McCall, "Induction of Protection Against Brugia Malayi Infection in Jirds By Microfilarial Antigens", Jour. Immun., 136, 1-5, (1986) concern similar induction of protection against Brugia Malayi infection in jirds by purified microfilarial antigens

## SUMMARY OF THE INVENTION

It is accordingly an object of the present invention to provide a preventive, rather than a therapeutic, way of combatting infection by Schistosoma mansoni.

It is a further object of the present invention to provide a. vaccine which can impart immunity against infection by Schistosoma mansoni.

These and other objects and advantages can be realized in accordance with the present invention pursuant to which there is provided a vaccine comprising an antigen which induces resistance to infection by Schistosoma mansoni.

The present invention concerns a monoclonal antibody which identifies the site of theSchistosoma mansoni parasite and which binds to a 68,000 molecular weight antigen of Schistosoma mansoni.

Somewhat more specifically, from the spleen cells of Schistosoma mansoni infected mice and myeloma cells there is produced a hybridoma, i.e., murine hybridoma, cell line which produces a monoclonal antibody specific to a Schistosoma mansoni 68,000 molecular weight antigen. This monoclonal antibody is employed to isolate from Schistosoma mansoni an antigen specific thereto, and such antigen is employed as a vaccine for imparting immunity. Such vaccine comprising an effective amount of such antigen in substantially pure form and a physiologically acceptable diluent.

## BRIEF DESCRIPTION OF THE DRAWINGS

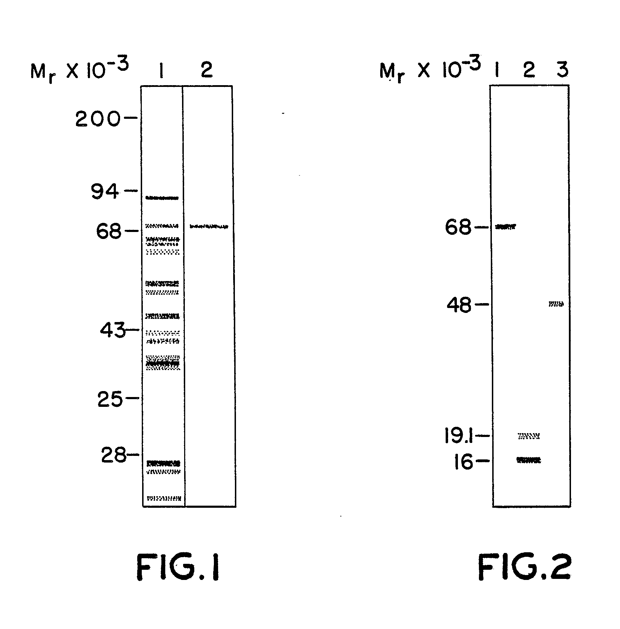

Fig. 1 represents the purification of an antigen according to the present invention (31-3B6) performed by immunoaffinity chromatography of soluble adult worm antigen ("SWAP") against an anti-schistosome murine monoclonal antibody according to the present invention (31-3B6 antibody) immobilized on Sepharose beads. Fig. 1 shows the results of an SDS-polyacrylamide gel electrophoresis performed on the recovered fractions. After silver staining of the gel, lane 1, containing non-binding parasite material, shows multiple protein bands. Lane 2, containing specifically bound material, shows a single band at 68,000 molecular weight. The relative migration of molcular weight standards is indicated at the left-hand border of Fig. 1.

Fig. 2 shows the results of immunoblotting performed against SWAP (lane 1), soluble egg antigen ("SEA") (lane 2), and schistosomula extract (lane 3), using rabbit antiserum raised against purified 31-3B6 antigen.

Fig. 3 is a plot representing cutaneous hypersensitivity to purified 31-3B6 antigen in mice immunized with 31-3B6 antigen and in control mice injected with PBS alone.

## DETAILED DESCRIPTION OF THE INVENTION

Specifically, immature Schistosoma mansoni worms are disrupted by repeat cycles of freezing and thawing and are injected into mice, advantageously along with an adjuvant to promote antibody production. After a few weeks the mice are sacrificed, their spleens removed, and the spleen cells fused with myeloma cells according to G. Kohler and C. Milstein, "Deviation of Specific Antibody-Producing Tissue Culture and Tumor Lines By Cell Fusion", European Journal of Immunology, Vol. 6, pages 511-519, (1976), producing hybridoma. The hybridoma are subdivided into wells and subjected to growth conditions. Clones are grown from single hybridoma cells; the resulting monoclonal antibodies, are then tested against Schistosoma mansoni to see which react therewith.

The monoclonal antibodies which are highly active against the parasite surface are independently expanded in quantity by injection into mice. The antibody is then taken from the mice, purified and fixed to a column of sepharose beads. Thereafter, ground Schistosoma mansoni extract is passed through the column whereby the held-antibody in turn holds those components of the Schistosoma mansoni extract recognizable thereby. Those components are thereafter eluted and the eluate constitutes the antigen or vaccine which, upon purification, can thereafter be used for immunization.

This active immunizing agent can thereafter be produced in large quantity, either through genetic engineering or by chemical synthesis after determining its amino acid configuration.

The active ingredient can be used for immunization in conventional manner, e.g., in saline solution possibly along with adjuvants and even other immunizing agents for protection against other infections.

The amounts of active material per injection of from 0.5 to 50 ml can vary widely and immunization can be achieved by a single injection or by multiple injections, some weeks apart.

The present invention affords the following advantages:

(1) successful immunization against metazoan parasites by inoculation with single parasite antigens can be conducted;

(2) a non-glycosylated peptide analog of the protective 68,000 molecular weight 31-3B6 antigen is recognized in expression products of schistosome cDNA clones and the polypeptides encoded by these clones are also identified by serum from infected humans. By contrast, Gryzch et al supra have reported their inability to antigenically identify a peptide analog of another protective schistosome glycoprotein (38,000 molecular weight) within the in vitro translation products of schistosome mRNA; and

(3) the 68,000 molecular weight antigen described herein appears to provide, without the use of adjuvants, a high level of acquired immunity in the mouse, a permissive host. The level of immunity to challenge infection is comparable to that produced with natural infection (A. Sher, P. MacKenzie and S. R. Smithers, J. Inf. Dis., 130, 626-633, (1974)), and well above levels of protection previously reported by Clegg and Smith, (using 155,000 and 53,000 molecular weight parasite antigens in alhydrogel adjuvant) and Hazdai et al, J. Immunol. 135, 2772-2780, (1985), (using 35,000 and 41,000 molecular weight antigens in Freund's adjuvant).

The monoclonal antibody (31-3B6) (ATCC HB 9597, deposited on December 2, 1987 with the American Type Culture Collection) of the present invention provided outbred mice with significant passive protection against challenge infection with Schistosoma mansoni. Like other protective monoclonal antibodies, 31-3B6 antibody reacts with antigens in three stages of the parasite life cycle, i.e., schistosomula, adult worms and eggs (D. A. Harn, M. Mitsuyama and J. R. David, "Anti-egg Monoclonal Antibodies Protect Against Cercarial Challenge In Vivo", J. Exp. Med., 159, 1371 (1984)).

Unlike previously described protective monoclonal antibodies, the 31-3B6 antibody recognizes a single 68,000 molecular weight antigen in adult worm extracts. By lectin binding and by chemical analysis, the immunoaffinity recovered 68,000 molecular weight antigen is a glycoprotein. Extensive periodic acid treatment failed to diminish 31-3B6 antibody binding to the purified antigen, indicating that the 31-3B6 monoclonal antibody likely binds to an epitope on the peptide portion of the antigen. Schistosomula incubated with 31-3B6 antibody had significantly greater mortality than larvae incubated with a control IgM. This finding suggests a direct toxic effect of the antibody on parasite survival, as has been described previously for sera obtained from infected rats (K. D. Murrell and B. Clay, "In Vitro Detection of Cytotoxic Antibodies to Schistosoma mansoni Schistosomules", Am. J. Trop. Med. Hyg., 21, 569, (1972)).

Active immunization with affinity purified 68,000 molecular weight antigen provided significant reductions in parasite burden after cercarial challenge. The 24-30% reductions in outbred CF1 mice and the 66% reduction in inbred Balb/C mice were obtained without the use of adjuvants. These results compare favorably with the results of other workers who have immunized hybrid mice with different purified parasite antigens combined with adjuvants. Smith and Clegg, supra achieved an average 25% reduction in worm burden by immunizing B6D2 F$_1$ mice with 2-10 µg of purified antigens (155,000 or 53,000 molecular weight) adsorbed with alum. Hazdai et al supra, achieved 34% increase in protection (above the level provided by Freund's adjuvant alone) by immunizing B6D2 F$_1$ mice with a 41,000/35,000 molecular weight dimeric antigen incomplete Freund's adjuvant. In studies using partially purified antigens, A. Sher, E. Pierce, S. Hieny and S. James, "Induction of Protective Immunity Against Schistosoma mansoni By a Non-Living Vaccine. IV. Fractionation and Antigenic Properties of a Soluble Adult Worm Immunoprophylactic Activity", J. Immunol., 136, 3878, (1986), have reported that inbred C57/black mice may obtain a 33-60% reduction in worm burden when coimmunized with M. bovis (BCG Strain) and a high molecular weight fraction of SWAP. Applicants obtained a similarly high level of protection (66%) in the susceptible Balb/C mouse using 31-3B6 antigen alone.

Down-regulation of cellular immune response plays an important role in limiting the level of disease which is associated with Schistosoma mansoni infection. It is important, therefore, to characterize the immune response induced by artificial immunization and assess its potential for increasing egg granuloma formation and consequent host tissue damage. The ELISA data herein indicate that 31-3B6 immunization results in a significant rise in antiparasite antibody levels after innoculation, but no enhancement (above control response) of antibody response after challenge infection. The results of the cutaneous hypersensitivity experiments reported herein indicate that immunization with the 31-3B6 antigen augments immediate (15 minutes) hypersensitivity, but not delayed-type (48 hours) hypersensitivity to subsequent antigen challenge. If a defined parasite vaccine is to succeed in protecting against Schistosoma mansoni, it should not enhance the level of immunopathology associated with infection. The studies of granuloma formation in uninfected mice reported herein indicate that 31-3B6 antigen immunization did not enhance granulomatous response to parasite eggs. This suggests that the 68,000 molecular weight antigen may be a good candidate antigen to attempt vaccination of previously infected animals, i.e., the antigen could potentially boost protective immune response in infected mice without exacerbating their cell-mediated tissue pathology.

Rabbit anti-idiotypic antibodies raised against 31-3B6 antibody may augment immune response to the 68,000 molecular weight antigen (M. Grzych, M. Capron, P. H. Lambert, C. Dissous, S. Torres and A. Capron, "An Anti-Idiotype Vaccine Against Experimental Schistosomiasis", Nature, 316, 74 (1985)). Immunization with anti-idiotypic antibodies alone (i.e., without 31-3B6 antigen) may also provide significant anti-parasite protection. An approach to improve immune response may involve the ability of deglycosylated antigen to provide active immunization against Schistosoma mansoni infection. If the peptide component of the 31-3B6 antigen provides significant protection, monospecific rabbit antiserum against the 31-3B6 antigen can be used to screen fusion proteins expressed by a recombinant cDNA library from Schistosoma mansoni. Immunoreactive fusion proteins may offer an extensive supply of cross-reacting antigen material for immunization trials at higher antigen doses. Furthermore, covalent linkage of fusion proteins to defined adjuvants such as trehalose dimycolate (cord factor) may provide significantly enhanced immune response to cross-reactive fusion protein antigens.

The method of preparing the hybridomas according to the present invention comprises the following steps:

(a) immunizing mice intraperitoneally with a freeze-thaw disrupted larvae of Schistosoma mansoni worms. The immunization schedule and immunogen concentration are such as to produce useful quantities of suitably primed splenocytes. Mice are immunized intraperitoneally (i.p.) with 0.1 ml of an emulsion prepared by mixing 4 mg/ml and an equal volume of complete Freund's adjuvant followed by four booster injections of the same doses using incomplete Freund's adjuvant at weekly intervals, followed by an intravenous (i.v.) boost using 0.1 mg ground Schistosoma mansoni worms in tris-saline 10 weeks later.

(b) Removing the spleens from the immunized mice and making a single cell suspension in an appropriate medium. These experimental techniques are well known.

(c) Fusing the suspended spleen cells with mouse mycloma cells from a suitable cell line by the use of suitable fusion promoter. The preferred ratio is about four spleen cells per myeloma cell. Many mouse myeloma cell lines are known and available, generally from members of the academic community or various deposit banks, such as the Salk Institute Cell Distribution Center, La Jolla, CA, U.S.A. A commonly use cell line class is 8-azaguanine resistant cell lines, which lack the enzyme hypoxanthine guanine phosphoribosyl transferase and hence will not be supported by HAT (hypoxanthine, aminopterin and thymidine) medium. It is also generally preferred that the myeloma cell line used by of the so-called "non-secreting" type, in that it does not itself produce any antibody, although secreting types may be used. In certain cases, however, secreting myeloma lines may be preferred. While the preferred fusion promoter is polyethylene glycol having an average molecular weight form about 1000 to about 4000 (commercially available as PEG 1000, etc.), other fusion promoters known in the art may be employed.

(d) Diluting and culturing in separate containers, the mixtures of unfused spleen cells, unfused myeloma cells and fused cells in a selective medium which will not support the unfused myeloma cells for a time sufficient to allow death of the unfused cells (about 14-16 days). The dilution may be a type of limiting one, in which the volume of diluent is statistically calculated to isolate a certain number of cells (e.g., 1-4) in each separate container (e.g., each well of a microtiter plate). A commonly used medium is HAT medium. Since the unfused spleen cells are non-malignant, they have only a finite number of generations. Thus, after a certain period of time (about 14-16 days) these unfused spleen cells fail to reproduce. The fused cells, on the other hand, continue to reproduce, because they possess the malignant quality of the myelome parent and the ability to survive in the selective medium of the spleen cell parent.

5

(e) Evaluating the supernatant in each container (well) for the presence of antibody to which identifies Schistosoma mansoni.

(f) Selecting (e.g., by limiting dilution) and cloning hybridomas producing the desired antibody.

Once the desired hybridoma has been selected and cloned, the resultant antibody may be produced in one of two ways. The purest monoclonal antibody is produced by in vitro culturing of the desired hybridoma in a suitable medium for a suitable length of time, followed by recovery of the desired antibody from the supernatant. The suitable medium and suitable length of culturing time are known or are readily determined. This in vitro technique produces monoclonal antibody, free from other antiparasite immune globulin. There is a small amount of other immune globulin present since the medium contains xenogeneic serum (e.g., fetal calf serum). However, this in vitro method may not produce a sufficient quantity or concentration of antibody form some purposes, since the concentration of monoclonal antibody is only about 50 $\mu$g/ml.

To produce a much greater concentration of slightly less pure monoclonal antibody, the desired hybridoma may be injected into mice, preferably syngenic or semi-syngenic mice. The hybridoma will cause formation of antibody producting tumors after a suitable incubation time, which will result in a high concentration of the desired antibody (about 5-20 mg/ml) in the peritoneal exudate (ascites) of the host mouse. Although these host mice also have normal antibodies in their blood and ascites, the concentration of these normal antibodies is only about 5% of the monoclonal antibody concentration.

Moreover, since these normal antibodies are not antiparasite in their specificity, the monoclonal antibody obtained from the harvested ascites or from the serum is essentially free of any contaminating antiparasite immune globulin. This monoclonal antibody is high titer and high ratio of specific to non-specific immune globulin.

The active component of the vaccine, i.e., the antigen, can be employed with a physiologically acceptable diluent (medium), e.g., phosphate buffered saline. Generally speaking, the antigen concentration in a physiologically acceptable medium will be between approximately less than 1 milligram and more than 10 micrograms per dose.

The vaccine can be administered by subcutaneous, intradermal or intramuscular injection. It is believed that the preferred administration route is subcutaneous injection. Generally speaking, the vaccine wil be administered in two doses about one month apart followed by a booster at six months to one year after primary immunization. The subsequent doses or the booster will depend on the level of antibody in the blood as a result of the initial immunization and in certain instances may be unnecessary.

The invention will be further described with reference to the following non-limiting examples.

## Examples

### Example 1: Preparation of Parasite Material

Schisotsome larvae and eggs were obtained from a life cycle maintained at Case Western Reserve University. Soluble adult worm antigen (SWAP) and soluble egg antigen (SEA) of S. mansoni were prepared as described in D. E. Colley, J. A. Cook, G. L. Freeman, R. K. Bartholomew and P. Jordan, "Immune Responses During Human Schistosomiasis Mansoni I. In Vitro Lymphocyte Blastogenic Response to Heterogeneous Antigenic Preparations From Schistosome Eggs, Worms and Cercariae", Int. Arch. Allergy Appl. Immunol., 53, 420, (1977); D. L. Boros and K. S. Warren, "Delayed Hypersensitivity-type Granuloma Formation and Dermal Reaction Induced and Elicited by a Soluble Factor Isolated from Schistosoma mansoni Eggs", J. Exp. Med., 132, 488, (1970).

Cercariae were transformed into schistosomula by in vitro penetration of isolated mouse skin (J. A. Clegg and S. R. Smithers, "The Effects of Immune Rhesus Monkey Serum on Schistosomula of Schistosoma mansoni During Cultivation In Vitro", Int. J. Parasitol, 2, 79, (1972)).

Schistosomula antigen extracts were prepared by incubating 20,000-40,000 three hour old schistosomula in 1% "TRITON X-100" detergent (Fisher Scientific Co, Fairlawn, New Jersey, U.S.A.) in 10 mm phosphate buffered saline ("PBS") (10 mm phosphate buffered normal saline solution) containing the protease inhibitor phenylmethylsulfonyl-fluoride (10mM) (Sigma Chemicals, St. Louis, Missouri, U.S.A.) for 30 minutes at 4° C. Antigen extracts were cleared of particular materials by ultracentrifugation (150,000 X g for 120 minutes) prior to use in immunoassays.

Example 2: Production of Monoclonal Antibodies

Female BALB/c mice (Jackson Laboratories, Bar Harbor, Maine) were immunized with an emulsion of 4000 schistosomula mixed 1:1 (vol:vol) with Freund's complete adjuvant (Difco, Detroit, Michigan, U.S.A.). Three weeks later, a second injection of 4000 schistosomula in Freund's incomplete adjuvant was administered to the mice subcutaneously. Two weeks later, the mice were bled and assayed for anti-SWAP titer by an ELISA (enzyme linked immunosorbent assay). ELISA-positive mice were given booster doses of 2000 disrupted schistosomula by tail vein and four days later the mice were sacrificed, their spleens removed, and splenocytes isolated (G. Kohler and C. Milstein, "Derivation of Specific Antibody-Producing Tissue Culture and Tumor Lines by Cell Fusion", Eur. J. Immuno., 6, 511, (1976)).

Supernatants of growing hybridoma clones were screened for antischistosome activity by ELISA against SWAP. Double immunodiffusion against monospecific antisera was used to identify the isotype of hybridoma antibodies (Research Products, Mount Prospect, Illinois).

Antibody activity against schistosomula was detected by indirect immunofluorescence assay with intact larvae. Supernatants of hybridoma clones were used as a source of antibody for some experiments, while for others the monoclonal antibody was obtained from malignant hybridoma ascites.

ELISA assays against SWAP and SEA were performed using alkaline-phosphatase conjugated sheep anti-mouse antiserum (New England Nuclear, Boston, Mass.) (A. M. Deelder, D. Kornelis, M. Makbin, H. N. Noordpool, R. M. Codfreid, J. P. Rotmans and B. F. Joostburg, "Applicability of Different Antigen Preparations in the Enzyme-Linked Immunosorbent Assay for Schistosomiasis Mansoni", Am. J. Trop. Med. Hyg., 29, 401, (1980).

Indirect immunofluorescence against schistosomula was performed using fluoresceinisothiocyanate-conjugated goat anti-mouse IgM antiserum according to the method of D. M. Zodda and S. M. Phillips, "Monoclonal Antibody-Mediated Protection Against Schistosoma mansoni Infection in Mice", J. Immunol., 129, 2326, (1982).


Example 3: In Vitro Killing Assay

In vitro Killing of Schistosomula (F. Santoro, P. J. Lachman, A. Capron and M. Capron, "Activation of Complement by Schistosoma mansoni Schistosomula: Killing of Parasites by the Alternative Pathway and Requirement of IgG for Classical Pathway Activation", J. Immunol., 123, 1551, (1979); C. A. Peck. M. D. Carpenter and A. A. F. Mahmoud, "Species-Related Innate Resistance to Schistosoma mansoni: Role of Mononuclear Phagocytes in Schistosomula Killing In Vitro", J. Clin. Invest, 71, 66, (1983)) was assessed in the presence and absence of monoclonal IgM. Antibody-enhanced complement-mediated killing and cell-mediated cytotoxicity were assessed using mouse peritoneal exudate cells and/or 10% fresh normal mouse serum. In cell killing assays, effector: target ratio was 4000 cells/larva. Parasite mortality was assessed at 18-24 hours by microscopic examination in the presence of 0.1% toluidine blue (A. Dessein, A. E. Butterworth, M. A. Vadas and J. R. David, "Maturation In Vivo of Schistosoma mansoni Schistosomula After Culture In Vitro with Granulocytes and Antibody", Infect. Immun., 39, 225, (1983); J. W. Kazura, M. M. Fanning, J. L. Blumer and A. A. F. Mahmound, "Role of Cell-Generated Hydrogen Peroxide in Granulocyte-Mediated Killing of Schistosomula of Schistosoma mansoni In Vitro", J. Clin. Invest., 67, 93, (1981)).


Example 4: Active Immunization

Active immunization was performed using immunoaffinity purified 31-3B6 antigen prepared from SWAP. Immunized mice received 0, 0.5 or 1 μg of antigen in PBS subcutaneously on day one and again on day fifteen of the protocol. Percutaneous cercarial challenge (100 or 500 larvae/mouse) was given on day twenty eight. Protection was assessed by recovery of developing larvae in the portal circulation at eight weeks after the challenge as described by S. R. Smithers and R. J. Terry, "The Infection of Laboratory Hosts With Cercariae of Schistosoma mansoni and the Recovery of Adult Worms", Parasitology, 55, 695, (1985).

In experiments to determine the effects of adjuvant on immunization with 31-3B6 antigen, animals were immunized with PBS alone (control), 31-3B6 antigen + PBS (1:1 vol: vol), 31-3B6 antigen with complete Freund's adjuvant (mixed 1:1), Freund's adjuvant + PBS (1:1), 31-3B6 antigen absorbed to alum (M. A. Smith, J. A. Clegg, D. Snary and A. J. Trajdosieicz, "Passive Immunization of Mice Against Schistosoma mansoni with IgM Monoclonal Antibody", Parasitology, 84, 83, (1982)) or alum/PBS. Each animal was subsequently boosted with the same material two weeks later and challenged with cercariae four weeks after initial injection.

## Example 5: Purification of 31-3B6 Antigen

Immunoaffinity columns were prepared by covalent linkage of IgM monoclonal to agarose (CNBr Sepharose, Pharmacia, Piscataway, New Jersey, U.S.A.) (M. A. Smith and J. A. Clegg, "Vaccination Against Schistosoma mansoni With Purified Surface Antigens", Science, 277, 535, (1985)). High titer IgM was prepared by hybridoma ascites fluid obtained by injecting $10^8$ cells into the peritoneal cavities of BALB/c mice pretreated with 2, 6, 10, 14 tetramethyldecane (T. T. McKearn, "Method for Growing Hybridomas in Rats and Mice", in R. Kennett, T. T. McKearn and K. B. Bechtol (Eds.), Monoclonal Antibodies, Plenum Press, N.Y., p. 403, (1980)). Antibody material was then purified by ammonium sulfate precipitation (35% saturation). For immunoaffinity chromatography, SWAP was first exposed to a control IgM/sepharose column for 30 minutes at 4°C. Unbound material from the control column was then incubated for a similar period on a column containing linked beads with 31-3B6 antibody. After washing with neutral and acid buffers, specifically bound parasite material eluted with 2M KSCN. The identity of eluted material was analysed by SDS-polyacrylamide electrophoresis (U. K. Laemmli, "Cleavage Structural Proteins During the Assembly of the Head of Bacteriophage T4", Nature, 227, 680, (1970), by Coomassie Blue-based protein assay (Biorad, Richmond, California), by anthrone reagent assay for hexose sugars (W. F. Durham, W. L. Bloom, G. Nature, T. Lewis and E. E. Mandel, "Rapid Measurement of Carbohydrate in Blood", Pub. Health Reports (U.S.), 65, 670 (1950) and by analysis of antigen binding to lectins covalently bound to sepharose beads (S. Lustigman, A. A. F. Mahmoud and J. Hamburger, "Glycopeptides in Soluble Egg Antigen of Schistosoma mansoni: Isolation, Characterization and Elucidation of Their Immunochemical and Immunopathological Relation to the Major Egg Glycoprotein", J. Immunol., 134, 1961, (1985)).

Specificity of 31-3B6 antibody for the protein component of its 68,000 molecular weight antigen was assessed according to the method of M. P. Woodward, W. W. Young, Jr. and R. A. Bloodgood, "Detection of Monoclonal Antibodies Specific for Carbohydrates Epitopes Using Periodate Oxidation, J. Immunol. Methods, 78, 143, (1985). By this technique, sugar residues on the molecule were destroyed through incubation with concentrations of periodate ranging from 5 to 160 mM for one hour prior to ELISA assay. See the results in Fig. 1.

## Example 6: Preparation of Polyclonal Rabbit Antiserum Against 31-3B6 Antigen

Two New Zealand white rabbits were immunized x2 at two week intervals with 50 μg of purified 31-3B6 antigen in Freund's complete adjuvant. Antigen was given in divided doses to axillary and inguinal regions. One week after the second injection, the animals were boosted with 50 μg of antigen in Freund's incomplete adjuvant delivered subcutaneously. The animals were bled on the third, fourth, and seventh days following final immunization. The animals were sera pooled, screened for anti-SWAP antibody and stored in aliquots at -70°C.

## Example 7: Immunoblotting

Following the procedure of H. Towbin, T. Staehlin and J. Gordon, "Electrophoretic Transfer of Proteins from Polyacrylamide Gels to Nitrocellulose Sheets: Procedure and Some Applications", Proc. Natl. Acad. Sci., U.S.A., 76, 4350, (1979) immunoblotting against SWAP, SEA and schistosomula extract was performed using both the immune rabbit antiserum described above and preimmune rabbit serum as control.

In Fig. 2, a single, 68,000 molecular weight band was identified in SWAP, whereas in schistosomula extract, both 68,000 and 48,000 molecular weights bands appeared. By contrast, in SEA, only low molecular weight components of 19,100 and 16,000 molecular weights were recognized. Apparent molecular weights of the identified bands are indicated on the left-hand margin of the blot. Control studies (not shown) indicated no recognition of parasite materials by preimmune rabbit serum.

## Example 8: Passive Protection

Passive protection was conferred on uninfected CF1 mice by injection with ten-fold concentrated hybridoma supernatant containing 31-3B6. The supernatant was prepared by ultra-filtration (filter PM10, Amicon Corp., Danvers, Mass., U.S.A.). Each mouse received 1 ml of concentrated supernatant intraperitoneally (i.p.) 24 hours prior to skin exposure to 500 cercariae (S. R. Smithers and R. J. Terry, Parasitology, 55, 695-700, 1965)). Booster doses of 0.5 ml concentrated supernatant were administered i.p. to each mouse immediately following recovery from anesthesia and again 24 hours later. After percutaneous challenge with cercariae (S. R. Smithers and R. J. Terry, "The Infection of Laboratory Hosts With Cercariae of Schistosoma Mansoni and the Recovery of adult Worms", Parasitology, 55, 695, (1965) protection was assessed by recovery of migrating lung stage shistosomula five days after challenge (A. Sher, P. Mackenzie and S. R. Smithers, "Decreased Recovery of Invading Parasites from the Lungs as Parameter of Acquired Immunity to Schistosomiasis in the Mouse", J. Inf. Dis., 130, 626-633, (1974)).

Example 9: Assays of Immune Response in Vaccinated Mice

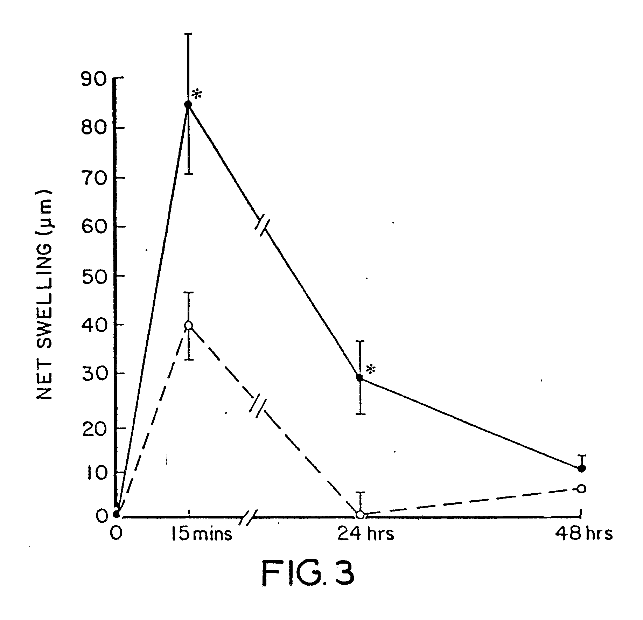

Cutaneous hypersensitivity was measured by injecting 0.5 μg or 31-3BG antigen material into the footpads of immunized and unimmunized CF₁ mice. Control injections of PBS were administered into the footpad of the contralateral paw and swelling in each paw measured with a micrometer. Results are reported in Fig. 3 as a net swelling (experimental side minus control side) for each mouse as described by Boros and Warren, supra. Egg granuloma response was measured in the lungs of immunized and unimmunized CF1 mice five days after tail vein injection of 500 eggs (F. VonLichtenberg, "Host Response to Eggs of S. mansoni I. Granuloma Formation in the Unsensitized Laboratory Mouse", Am J. Pathol., 41, 711, (1962); A. A. F. Mahmoud, M. A. Mandel, K. S. Warren and L. T. Webster, Jr., "Niridazole II. A Potent Long-Acting Suppressant of Cellular Hypersensitivity", J. Immunol., 114, 279, (1974)).

Twenty-eight days after the first injections, animals were challenged with 0.5 μg of antigen in one hind footpad and an equivalent volume of PBS in the contralateral paw.

Circulating antibody levels during the course of immunization and subsequent challenge infection were measured on individual serum samples at two standard dilutions by ELISA assay against SWAP (1.5 μg/well) and purified 31-3B6 antigen (1.5 μg/well).

The differences between experimental groups were analyzed statistically using Student's t-test for unpaired data.

Net swelling of the antigen paw was significantly greater in immunized mice (closed circles in Fig. 3) than in control mice (open circles in Fig. 3) at 15 minutes and 24 hours after footpad injection (P less than 0.01 and 0.005, respectively). There was no significant difference in footpad swelling at 48 hours.

## RESULTS

## 1. Antibody Development and Characterization

After BALB/c mice were immunized with freeze thaw disrupted schistosomula in Freund's adjuvant, splenocytes from those immunized mice with anti-schistosome titers of > 1:100 were fused with P3NS1 myeloma cells. Six SWAP-positive, SEA-positive hybridoma clones were screened for reactivity against schistosomula. The monoclonal 31-3B6 was chosen because of its linear reactivity with the total schistosomulum surface of IFA. The antibody isotype produced by 31-3B6 clone was determined to be IgM.

## 2. Effects of 31-3B6 Antibody on Schistosomula Survival In Vitro and In Vivo

In vitro culture of schistosomula in 31-3B6 supernatant was found to reduce larval survival significantly. The survival rate of 100 schistosomula in overnight culture in 300 μl of control supernatant (containing irrelevant IgM monoclonal antibody) was 88 ± 2%. By contrast, survival in 31-3B6 supernatant was found to be 68 ± 2% (P<0.001, mean ± SE for 6 experiments). Further experiments showed that killing by 31-3B6 supernatants, even concentrated up to 10-fold, was not enhanced by complement or by antibody-dependent cell-mediated toxicity using murine peritoneal exudate cells (data not shown).

In a screening (lung recovery) assay of larval infection, passive protection was conferred on uninfected CF1 mice by injection with ten-fold-concentrated hybridoma supernatant containing 31-3B6 antibody. In two separate experiments, the mean parasite recovery in 31-3B6 treated animal was 27% lower than in animals injected with irrelevant IgM (a mean ± SE of 120 ± 10 schistosomula/mouse for 31-3B6 treated animals versus 165 ± 11 schistosomula/mouse for animals treated with control IgM P<0.01 (see Table 1 hereinbelow).

### 3. Isolation and Characterization of 31-3B6 Antigen

Immunoaffinity columns prepared with purified 31-3B6 antibody reproducibly and specifically bound a single 68,000 molecular weight antigen (see Fig. 1). By immunoblotting, rabbit polyclonal antiserum raised against the 31-3B6 antigen recognized a 68,000 molecular weight antigen in SWAP and in detergent extracts of 3 hour old schistosomula (see Fig. 2). In addition, a second antigen band of 48,000 molecular weight was detected in schistosomula extract. The antigens bound in SEA were found to be multiple and of lower molecular weight, suggesting the presence of cross reacting antigenic epitopes in egg material. By colorimetric assays, the immunoaffinity-purified 68,000 molecular weight antigen proved to be 25% hexose and 75% protein by weight. The glycoprotein nature of this antigen was confirmed by evidence of specific, reversible binding to concanavalin A-sepharose, wheat germ agglutinin-sepharose, and peanut lectin sepharose (data not shown). In ELISA assays, it was noted that periodate digestion of the carbohydrate components of the antigen did not reduce 31-3B6 antibody binding: $A_{405}$ of 125 $\mu$g 31-3B6 antibody binding to two $\mu$g/well of purified antigen was 0.089 ± .001. For antigen preincubated with 5 $\mu$M and 160 $\mu$M periodate for one hour, then reacted with 31-3B6 antibody, the mean absorbances were 0.088 ± .016 and 0.165 ± .009 respectively.

Active immunization with 31-3B6 antigen was obtained by injecting normal CF1 mice with immunoaffinity purified antigen in PBS. Control animals received PBS alone. As detailed in Table 2 hereinbelow, significant protection of immunized animals was demonstrated in each of four separate experiments. In CF1 mice, protection ranged from 24 to 30%, with the average decrease in parasite burden being 26%. In inbred BALB/c mice, protection was found to be 66% (P<0.001).

Experiments were undertaken to test the ability of common adjuvants to enhance the immunity provided by 31-3B6 antigen. Results of worm recovery for each group are summarized in Table 3 hereinbelow. Significant reduction in parasite recovery was seen for the groups receiving 31-3B6 antigen alone or 31-3B6 antigen mixed with Freund's adjuvant. Of note, the addition of Freund's adjuvant offered no significant change in the level of protection conferred by the antigen. By contrast, it was found that mice receiving antigen mixed with alum had an intermediate level of larval recovery (27 ± 5 larvae/mouse as compared to 32 ± 3 for control animals and 24 ± 2 for 31-3B6-immunized mice). This recovery of larvae from alum + antigen immunized mice was not significantly different form either control or antigen-immunized groups.

### 4. Characterization of Immune Response in Immunized Mice

The antiparasite antibody levels of immunized mice rose significantly during the course of immunization, peaking at seven days after the first inoculation (see Table 4 hereinbelow). Following cercarial challenge, both immunized mice and unimmunized mice had significant increases in anti-antigen antibody levels during the course of infection.

$CF_1$ mice immunized with purified 31-3B6 antigen had significantly greater immediate footpad swelling in response to 31-3B6 antigen than did unimmunized mice (p< 0.01) (see Fig. 3). There was, however, no significant difference between control and immunized mice in footpad swelling response at 48 hours after injection.

To assess whether immunization could significantly alter the immunopathology of S. mansoni infection, egg granuloma formation was measured in unimmunized mice and in mice immunized with 31-3B6 antigen. Mice were challenged intravenously with 500 S. mansoni eggs and their circumoval granulomatous response (in the lung tissue) was measured eight days after challenge. In the six control $CF_1$ mice, the mean circumoval granuloma size was 12167 ± 1902 $\mu m^2$ (mean ± SE) whereas in six mice preimmunized with 1 $\mu$g 915 $\mu m^2$ (difference from control, p<0.05).

TABLE 1

| Passive Immunization with Antibody 31-3B6[a] | | | |
|---|---|---|---|
| Reduction | Antibody Given | Schistosomula Recovered | |
| Experiment 1 | Control IgM | 148 ± 7 | |
| | 31-3B6 | 104 ± 5* | 30% |
| Experiment 2 | Control IgM | 182 ± 16 | |
| | 31-3B6 | 136 ± 5* | 25% |

[a]Uninfected CF1 mice were given 3 doses of 10 fold concentrated 31-3B6 supernatant 24 hours prior to and 1 hour and 24 hours following cutaneous exposure to 500 cercariae of S. mansoni k>//UND\. Developing larvae were quantified by recovery from the lung at 5 days after challenge.
* Significant difference (p<0.05).

TABLE 2

Active Immunization of CF1 and BALB/c Mice Against Schistosomiasis
Mansoni Using Immunoaffinity Purified 68,000 MW antigen [a]

| Experiment No. | Dose of antigen | n | Number of schistosomes recovered | Protection |
|---|---|---|---|---|
| 1 | 0 | 5 | 158 ± 6 | |
| | 1 μg | 3 | 112 ± 11[*] | 30% |
| 2 | 0 | 8 | 35 ± 3 | |
| | 1 μg | 10 | 27 ± 1[*] | 24% |
| 3 | 0 | 10 | 32 ± 3 | |
| | 1 μg | 10 | 24 ± 2[*] | 25% |
| 4 | 0 | 10 | 70 ± 4 | 66% |
| | 0.5 μg | 10 | 23 ± 3[+] | |

[a] Purified 68,000 molecular weight antigen was prepared from SWAP by immunoaffinity chromatography as detailed in Fig. 2. Various doses of antigen in 0.15 ml saline were administered subcutaneously to the dorsal skin of uninfected CF1 or BALB/c mice. Control mice received saline injection alone. An equivalent booster immunization was given 2 weeks later and on Week 4; all animals were challenged by shaved abdominal skin exposure to 500 or 100 cercariae of S. mansoni. Protection was assessed by recovery of developing larvae in the portal circulation of infected mice at 8 weeks after exposure.

[*] Significant difference from control group p<0.05. Student's t-test.

[+] Significant difference from control group p<0.001

TABLE 3

| Adjuvant Effects on Immunization Against S. Mansoni with 31-3B6 Antigen[a] | | | |
|---|---|---|---|
| Immunogen | n | Number of Schistosomes Recovered | Protection |
| Control (PBS)[b] | 10 | 32 ± 3 | --- |
| 31-3B6 antigen[b] | 10 | 24 ± 2* | 25% |
| Freund adjuvant + PBS | 6 | 29 ± 4 | 9% |
| 31-3B6 + Freund Adjuvant | 6 | 24 ± 4* | 25% |
| Alum + PBS | 6 | 36 ± 3 | --- |
| 31-3B6 + Alum | 6 | 27 ± 5 | 16% |

[a]CF$_1$ mice were injected subcutaneously with PBS or 1 μg 31-3B6 antigen with and without adjuvants on day 1 and again on day 15, and challenged with 100 cercariae on day 28. Worm recovery was determined by portal perfusion 8 weeks after challenge.

[b] Same as experiment 3, Table 2.

* Significant difference from control ($p<0.05$)


TABLE 4

| Murine Antibody Response to Immunization with 31-3B6 Antigen and/or Cercarial Infection | | | |
|---|---|---|---|
| Mouse Strain | Antigen Exposure[a] | Antibody Response[b] | |
| | | (ELISA ASSAY) | |
| | | Anti 31-3B6 | Anti-SWAP |
| | | (1:1000 Dilution) | (1:1000 Dilution) |
| BALB/c | NIL | 0.044 ± .006 | 0.130 ± .057 |
| | 0.5 μg 31-3B6 (1 week) | 0.240 ± .027* | 0.280 ± .093 |
| | 100 cercariae (1 week) | 0.610 ± .219* | 0.490 ± .123 |
| | Antigen + Cercariae | 0.400 ± .053* | 0.405 ± .111* |
| | | (1:100 Dilution) | (1:100 Dilution) |
| CF1 | NIL | 0.236 ± .009 | 0.098 ± .012 |
| | 1.0 μg 31-3B6 (1 week) | 0.499 ± .063* | not tested |
| | 100 cercariae (1 week) | 0.997 ± .085* | 0.850 ± .070 |

[a]The respective samples were obtained either prior to immunization (= NIL), 1 week after immunization with purified antigen, 1 week after challenge infection of unimmunized mice or 1 week after challen e infection of immunized mice.

[b]Measured as net A$_{405}$ for ELISA assay against 31-3B6 OR SWAP 1.5 μg/well) at serum dilutions indicated, results are reported as mean ± SE for 5 individuals in each group.

* Significantly greater than control (NIL) $p<0.05$.


It will be understood that the specification and examples are illustrative but not limitative of the present invention and that other embodiments within the spirit and scope of the invention will suggest themselves to those skilled in the art.

## Claims

1. A monoclonal antibody which binds with a 68,000 molecular weight antigen of Schistosoma mansoni.

2. A monoclonal antibody of claim 1, which is secreted by a murine hybridoma.

3. A monoclonal antibody of claim 1, said antibody being produced by a hybridoma cell line having the identifying characteristics of hybrodima cell line ATCC HB 9597.

4. A vaccine for immunization against Schistosoma mansoni infection comprising an effective amount of a substantially pure antigen which is identified by the monoclonal antibody according to claim 1, and a physiologically acceptable diluent.

5. A hybridoma cell line ATCC HB 9597.

14

FIG.1

FIG.2

FIG. 3

EP 0 318 879 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | THE JOURNAL OF IMMUNOLOGY, vol. 139, no. 12, 15th December 1987, pages 4218-4224, The American Association of Immunologists, US; C.H. KING et al.: "Isolation and characterization of a protective antigen for adjuvant-free immunization against Schistosoma mansoni" * Abstract * --- | 1-5 | A 61 K 39/395 C 12 P 21/00 |
| X | CLINICAL RESEARCH, vol. 34, no. 2, 1986, page 675; R.E. BLANTON et al.: "Molecular cloning of a schistosoma mansoni candidate vaccine antigen" * Abstract * --- | 1-5 | |
| X | CLINICAL RESEARCH, vol. 34, no. 2, 1986, page 522A, C.H. KING et al.: "Isolation and characterization of a M 68,000 candidate vaccine antigen for Schistosomiasis mansoni" * Abstract * --- | 1-5 | **TECHNICAL FIELDS SEARCHED (Int. Cl.4)** |
| A,D | THE JOURNAL OF IMMUNOLOGY, vol. 135, no. 4, October 1985, pages 2772-2779, The American Association of Immunologists, US; R.T. HAZDAI et al.: "Protective monoclonal antibody against Schistosoma mansoni: antigen isolation, characterization, and suitability for active immunization" ----- | 1-5 | A 61 K C 12 P |

The present search report has been drawn up for all claims

| Place of search | Date of completion of the search | Examiner |
|---|---|---|
| THE HAGUE | 16-02-1989 | TURMO Y BLANCO C.E. |